# EUROPEAN PATENT APPLICATION

(11) **EP 2 226 323 A1**
(43) Date of publication of application: **08.09.2010**
(21) Application number: 09382025.6
(22) Date of filing: 27.02.2009
(51) Int. Cl.: C07D 471/04, A61K 31/4745, A61P 37/00, A61P 17/06, A61P 19/02

(54) **New tetrahydropyrazolo[3,4-c]isoquinolin-5-amine derivatives**

(71) Applicant: Almirall, S.A., 08022 Barcelona (ES)
(72) Inventor: Taltavull Moll, Joan, 08015 Barcelona (ES); Gracia Ferrer, Jordi, 08720 Vilafranca del Penedès, (Barcelona) (ES); Pages Santacana, Lluis Miquel, 08029 Barcelona (ES)
(74) Representative: Elzaburu Marquez, Alberto

(57) **Abstract**

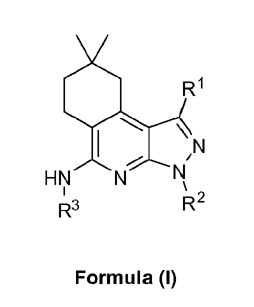

New tetrahydropyrazolo[3,4-c]isoquinolin-5-amine derivatives having the chemical structure of formula (I) are disclosed; as well as process for their preparation, pharmaceutical compositions comprising them and their use in therapy as inhibitors of the phosphodiesterase IV (PDE4).

## Description

The present invention relates to new therapeutically useful tetrahydropyrazolo[3,4-c]isoquinolin-5-amine derivatives, to processes for their preparation and to pharmaceutical compositions comprising them. These compounds are potent and selective inhibitors of phosphodiesterase 4 (PDE4) and are thus useful in the treatment, prevention or suppression of pathological conditions, diseases and disorders known to be susceptible of being improved by inhibition of PDE4.

Phosphodiesterases (PDEs) comprise a superfamily of enzymes responsible for the hydrolysis and inactivation of the second messenger cyclic adenosine monophosphate (cAMP) and cyclic guanosine monophosphate (cGMP). Eleven different PDE families have been identified to date (PDE1 to PDE11) which differ in substrate preference, catalytic activity, sensitivity to endogenous activators and inhibitors, and encoding genes.

The PDE4 isoenzyme family exhibits a high affinity for cyclic AMP but has weak affinity for cyclic GMP. Increased cyclic AMP levels caused by PDE4 inhibition are associated with the suppression of cell activation in a wide range of inflammatory and immune cells, including lymphocytes, macrophages, basophils, neutrophils, and eosinophils. Moreover, PDE4 inhibition decreases the release of the cytokine Tumor Necrosis Factor α (TNFα). The biology of PDE4 is described in several recent reviews, for example M. D. Houslay, P. Schafer, K. Y. Zhang, Drug Discov Today 2005, 10, 1503-19.

In view of these physiological effects, PDE4 inhibitors of varied chemical structures have been recently disclosed for the treatment or prevention of chronic and acute inflammatory diseases and other pathological conditions, diseases and disorders known to be susceptible to amelioration by inhibition of PDE4. See, for example, US 5449686, US 5710170, WO 98/45268, WO 99/06404, WO 01/57025, WO 01/57036, WO 01/46184, WO 97/05105, WO 96/40636, WO03/097613, US 5786354, US 5773467, US 5753666, US 5728712, US 5693659, US 5679696, US 5596013, US 5541219, US 5508300, US 5502072 or H. J. Dyke and J. G. Montana, Exp. Opin. Invest. Drugs 1999, 8, 1301-1325.

A few compounds having the capacity to selectively inhibit phosphodiesterase 4 are in active development. Examples of these compounds are roflumilast, GSK-256066, apremilast, tetomilast, rolipram, MK-0873 and oglemilast.

It is known that the clinical development in human of early PDE4 inhibitors such as rolipram has been hampered by the appearance of side effects such as nausea and vomiting at therapeutic plasma levels (Curr. Pharm. Des. 2002, 8, 1255-96). The compounds described in the present invention are potent and selective PDE4 inhibitors with reduced emetic potential. This property makes them useful for the treatment or prevention of pathological conditions or diseases such as respiratory diseases, skin disease, inflammatory diseases, diseases of the central or peripheral nervous system and cancer. These diseases include but are not limited to asthma, chronic obstructive pulmonary disease, allergic rhinitis, rheumatoid arthritis, multiple sclerosis, atopic dermatitis, psoriasis and inflammatory bowel disease.

PDE4 inhibitors, such as the compounds of the present invention referred to below, can also be used in combination with other drugs known to be effective in the treatment of these diseases. For example, they can be used in combination with bronchodilators such as β2-adrenergic agonists, antagonists of M3 muscarinic receptors or dual acting molecules combining β2 agonism with M3 antagonism, anti-allergics such as anti-histamines, mast cell stabilizers, CRTH antagonists, anti-inflammatory agents such as corticosteroids, LTD4 receptor antagonists, leukotriene synthesis inhibitors, COX inhibitors and PDE inhibitors, immunosuppressive agents such as calcineurin inhibitors, cyclosporin A, rapamycin, T-cell receptor blockers, B cell receptor blockers, and/or antiinfectives such as antibiotics, antimycotics, antiviral agents.

Accordingly, the present invention provides a compound of formula (I) or a tautomer thereof: 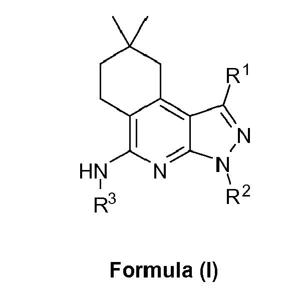 wherein:
- R¹ is selected from the group consisting of a hydroxy group and an amino group,
- R² is selected from the group consisting of a hydrogen atom, a linear or branched C₁₋₄ alkyl group, -(CH₂)₍₀₋₄₎-C(O)NR^{a}R^{b}, a C₁₋₄ alkyl-C₅₋₁₀ aryl group, a C₁₋₄ alkyl-5 to 10 membered N-containing heteroaryl group, a C₁₋₄alkyl-5 to 10 membered N-containing heterocyclic group, a sulfonyl-C₅₋₁₀ aryl group and a sulfonyl-C₁₋₄ alkyl group,
wherein the aryl, heteroaryl and heterocyclic groups are optionally substituted by one or more substituents selected from the group consisting of a C₁₋₄ alkyl group, a C₁₋₄ alkoxy group, a hydroxy, a halogen atom, a trifluoromethyl group, a carboxy group, a C₁₋₄ alkoxycarbonyl group, a thio-C₁₋₄ alkyl group, a sulfonyl-C₁₋₄ alkyl group, a cyano group, a carbamoyl group, -NR^{a}R^{b} and -C(O)NR^{a}R^{b};
wherein R^{a} and R^{b} independently represent a hydrogen atom or a C₁₋₄ alkyl group; and
- R³ is selected from the group consisting of a C₅₋₁₀ aryl group and a 5 to 10 membered heteroaryl group containing at least one heteroatom selected from N, S and O, wherein the aryl and the heteroaryl group are optionally substituted by one or more substituents selected from halogen atoms, C₁₋₄ alkyl groups and C₁₋₄ alkoxy groups,
or a pharmaceutically acceptable salt or N-oxide thereof.

Further objectives of the present invention are to provide compounds of formula (I) for use in the treatment of a pathological condition or disease susceptible to amelioration by inhibition of phosphodiesterase IV, such as asthma, chronic obstructive pulmonary disease, allergic rhinitis, rheumatoid arthritis, multiple sclerosis, atopic dermatitis, psoriasis or inflammatory bowel disease.

The invention is also directed to
- a pharmaceutical composition comprising a compound as defined above in association with a pharmaceutically acceptable diluent or carrier;
- use of a compound as defined above for the manufacture of a medicament for the treatment of a pathological condition or disease as defined above;
- a method for treating a subject afflicted with a pathological condition or disease as defined above, which comprises administering to said subject an effective amount of a compound as defined above; and
- a combination product comprising (i) a compound according to anyone of claims 1 to 10, and (ii) one or more active ingredients selected from the group consisting of β2-adrenergic agonist, anti-cholinergic, an anti-allergic agent, an anti-inflammatory agent, an immunosuppressant, and an anti-infective agent, for simultaneous, separate or sequential use in the treatment of the human or animal body.

As used herein the term C₁₋₄ alkyl embraces optionally substituted, linear or branched hydrocarbon radicals having 1 to 4 carbon atoms. Preferably, C₁₋₄ alkyl groups are unsubstituted. Examples include methyl, ethyl, *n*-propyl, *i*-propyl, *n*-butyl, *sec*-butyl and *tert*-butyl radicals.

As used herein the term C₁₋₄ alkoxy embraces optionally substituted, linear or branched oxygen containing radicals each having 1 to 4 carbon atoms. Preferably, C₁₋₄ alkoxy groups are unsubstituted. Examples include methoxy, ethoxy, *n*-propoxy, *i*-propoxy, *n-*butoxy, *sec*-butoxy and *tert*-butoxy radicals.

As used herein, the term C₁₋₄ alkoxycarbonyl group embraces radicals of formula - C(O)O(C₁₋₄ alkyl), wherein said C₁₋₄alkyl is an optionally substituted, linear or branched hydrocarbon radical having 1 to 4 carbon atoms. Preferably, C₁₋₄ alkoxycarbonyl groups are unsubstituted. Examples include methoxycarbonyl, ethoxycarbonyl, n-propyloxycarbonyl, *i*-propyloxycarbonyl, *n*-butyloxycarbonyl, *sec*-butyloxycarbonyl and *tert*-butyloxycarbonyl radicals.

As used herein, the term C₅₋₁₀ aryl group typically embraces optionally substituted C₅-C₁₀, preferably C₆-C₁₀, monocyclic or polycyclic aryl radicals such as phenyl, naphthyl, anthranyl and phenanthryl. Phenyl is preferred. A said optionally substituted aryl is typically unsubstituted or substituted with 1, 2 or 3 substituents which may be the same or different. Unless otherwise specified, the substituents on an aryl group are typically themselves unsubstituted.

As used herein, the term 5 to 10 membered heteroaryl group typically embraces optionally substituted 5- to 10- membered ring systems comprising at least one heteroaromatic ring and containing at least one heteroatom selected from O, S and N. A said optionally substituted heteroaryl group is a single ring or two or more fused rings, wherein at least one ring contains a heteroatom. A said optionally substituted heteroaryl group is typically unsubstituted or substituted with 1, 2 or 3 substituents which may be the same or different. When a heteroaryl group carries 2 or more substituents, the substituents may be the same or different. Unless otherwise specified, the substituents on a heteroaryl group are typically themselves unsubstituted.

Preferred 5 to 10 membered heteroaryl groups are 5 to 6 membered heteroaryl groups. Examples of 5 to 10 membered heteroaryl groups include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, triazolyl and pyrazolyl. Pyridyl is particularly preferred.

As used herein, the term 5 to 10 membered N-containing heteroaryl group typically embraces a 5- to 10- membered ring system, comprising at least one heteroaromatic ring and containing at least one N atom. Said 5 to 10 membered N-containing heteroaryl group is a single ring (monocyclic) or two or more fused rings (polycyclic), wherein at least one ring contains an N atom.

Preferred 5 to 10 membered N-containing heteroaryl groups are 5 to 6 membered N containing heteroaryl groups. Examples of 5 to 10 membered N-containing heteroaryl groups include pyridyl, pyrazinyl, pyrimidinyl and pyridazinyl. Pyridyl is preferred.

As used herein, the term 5 to 10 membered N-containing heterocyclic group typically embraces a non-aromatic, saturated or unsaturated C₅-C₁₀ carbocyclic ring system, such as a 5, 6 or 7 membered radical, in which one of the carbon atoms is replaced by N and optionally one or more, for example 1, 2, 3 or 4, preferably 1 or 2, further carbon atoms are replaced by one or more futher heteroatoms selected from N and O, preferably O. Thus, preferably, said 5 to 10 membered N-containing heterocyclic group typically contains 1 nitrogen atom and 0 or 1 oxygen atoms. Saturated heterocyclic rings are preferred.

Preferred 5 to 10 membered N-containing heterocyclic groups are 5 to 6 membered N-containing heterocyclic groups. Examples include azetidyl, piperidyl, pyrrolidyl, pyrrolinyl, piperazinyl, morpholinyl, thiomorpholinyl and pirazolidinyl. Morpholinyl is a preferred example.

As used herein, the term halogen atom embraces chlorine, fluorine, bromine or iodine atoms typically a fluorine, chlorine or bromine atom. The term halo when used as a prefix has the same meaning.

When R² represents a C₁₋₄alkyl-C₅₋₁₀aryl group, a C₁₋₄alkyl-5 to 10 membered N-containing heteroaryl group or a C₁₋₄ alkyl-5 to 10 membered N-containing heterocyclic group, it is to be understood that the C₁₋₄ alkyl is preferably bonded to the nitrogen atom of the molecule as depicted in formula (I).

When R² represents a sulfonyl-C₅₋₁₀ aryl group or a suifonyl-C₁₋₄alkyl group it is to be understood that the S atom is preferably bonded to the nitrogen atom of the molecule as depicted in formula (I).

When the aryl, heteroaryl, or heterocyclic groups that R² represents are substituted with a thio-C₁₋₄ alkyl group or a sulfonyl-C₁₋₄ alkyl group, it is to be understood that the S atom is preferably bonded to the aryl, heteroaryl, or heterocyclic group. When the aryl, heteroaryl, or heterocyclic groups that R² represents are substituted with a C₁₋₄ alkoxycarbonyl group, it is to be understood that the carbonyl carbon of the alkoxycarbonyl group is bonded to the aryl, heteroaryl, or heterocyclic group.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

The present invention also includes tautomers of the compounds of formula (I). Typically, these tautomers have the formula (I'): 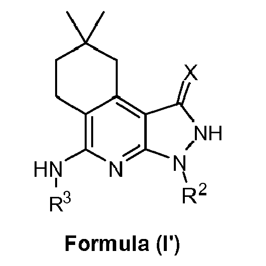 wherein X is O or NH. Preferably X is O.

Typically, R¹ represents a hydroxy group.

Typically, R² represents hydrogen atom, a C₁₋₄ alkyl-C₅₋₁₀ aryl group, a C₁₋₄ alkyl-5 to 10 membered N-containing heteroaryl group, a C₁₋₄ alkyl-5 to 10 membered N-containing heterocyclic group, wherein the aryl, heteroaryl and heterocyclic groups are optionally substituted by one or two substituents selected from the group consisting of C₁₋₄ alkoxy group, carboxy group, C₁₋₄ alkoxycarbonyl group, cyano group and carbamoyl group. Preferably, R² represents hydrogen atom, a C₁₋₄ alkyl-phenyl group, wherein the phenyl group is monosubstituted by a substituent selected from the group consisting of carboxy group, C₁₋₄ alkoxycarbonyl group, cyano group and carbamoyl group. More preferably, R² represents a C₁₋₄ alkyl-phenyl group, wherein the phenyl group is monosubstituted by a substituent selected from the group consisting of carboxy group, methoxycarbonyl group, cyano group and carbamoyl group.

Typically, R³ represents a 5 to 10 membered heteroaryl group optionally substituted by one or two substitutents selected from fluorine atoms, chlorine atoms and methyl groups. Preferably, R³ represents a pyridyl group optionally substituted with one or two substituents selected from chlorine atoms and methyl groups. More preferably, R³ represents a pyridyl group substituted by two chlorine atoms.

In a preferred embodiment of the present invention, R¹ represents a hydroxy group; R² represents a C₁₋₄ alkyl-phenyl group, wherein the phenyl group is monosubstituted by a substituent selected from the group consisting of carboxy group, methoxycarbonyl group, cyano group and carbamoyl group; and R³ represents a pyridine substituted by two chlorine atoms.

In another preferred embodiment of the present invention, R¹ represents a hydroxy group or a amino group; R² represents a hydrogen atom, a methyl group, a -CH₂CONH₂ group, a pyridine-3-yl-methyl group, a benzyl group optionally substituted by one substitutent selected from the group consisting of a methoxy group, a carboxy group, a methoxycarbonyl group, a carbamoyl group and a cyano group; a 3-phenylpropyl group optionally substituted by one substituent selected from the group consisting of a cyano group and carbamoyl group; a 3-morpholinopropyl group or a 3-(dimethylcarbamoyl)phenylsulfonyl group and R³ represents a 3,5-dichloropyridin-4-yl group.

Particular individual compounds of the invention include:
5-(3,5-Dichloropyridin-4-ylamino)-8,8-dimethyl-6,7,8,9-tetrahydro-3H-pyrazolo[3,4-c]isoquinolin-1-ol;
N⁵-(3,5-Dichloropyridin-4-yl)-8,8-dimethyl-6,7,8,9-tetrahydro-3H-pyrazolo[3,4-c]isoquinoline-1,5-diamine;
3-(5-(3,5-Dichloropyridin-4-ylamino)-1-hydroxy-8,8-dimethyl-6,7,8,9-tetrahydro-3H-pyrazolo[3,4-c]isoquinolin-3-ylsulfonyl)-N,N-dimethylbenzamide;
5-(3,5-Dichloropyridin-4-ylamino)-3-(3-methoxybenzyl)-8,8-dimethyl-6,7,8,9-tetrahydro-3H-pyrazolo[3,4-c]isoquinolin-1-ol;
5-(3,5-Dichloropyridin-4-ylamino)-3,8,8-trimethyl-6,7,8,9-tetrahydro-3H-pyrazolo[3,4-c]isoquinolin-1-ol;
Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid;
5-(3,5-Dichloropyridin-4-ylamino)-8,8-dimethyl-3-(pyridin-3-ylmethyl)-6,7,8,9-tetrahydro-3H-pyrazolo[3,4-c]isoquinolin-1-ol;
2-(5-(3,5-Dichloropyridin-4-ylamino)-1-hydroxy-8,8-dimethyl-6,7,8,9-tetrahydro-3H-pyrazolo[3,4-c]isoquinolin-3-yl)acetamide;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide;
Methyl 4-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate
4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzonitrile;
4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide;
5-(3,5-Dichloropyridin-4-ylamino)-8,8-dimethyl-3-(3-morpholinopropyl)-6,7,8,9-tetrahydro-3H-pyrazolo[3,4-c]isoquinolin-1-ol;
3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzonitrile;
3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzamide.

### Of outstanding interest are:

5-(3,5-Dichloropyridin-4-ylamino)-8,8-dimethyl-6,7,8,9-tetrahydro-3H-pyrazolo[3,4-c]isoquinolin-1-ol;
Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide;
4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzonitrile;
3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzamide.

According to another embodiment the present invention covers pharmaceutical compositions comprising one or more of the compounds of formula (I), as hereinabove described, in admixture with pharmaceutically acceptable diluents or carriers.

In still another embodiment the present invention covers a combination product comprising (i) a compound of formula (I), as hereinabove described, and (ii) another compound selected from (a) β2-adrenergic agonists, (b) anti-cholinergics such as antagonists of M3 muscarinic receptors (c) antiinflammatory agents, (d) anti-allergic agents, (e) immunosuppressants and (f) anti-infectives; for simultaneous, separate or sequential use in the treatment of the human or animal body.

According to still another embodiment of the present invention is directed to compounds of formula (I), as hereinabove described, for use in the treatment of the pathological condition or disease susceptible to amelioration by inhibition of phosphodiesterase 4 and also for the use of compounds of the present invention for the manufacture of a medicament for the treatment or prevention of a pathological condition or disease susceptible to amelioration by inhibition of phosphodiesterase 4. It is a preferred embodiment to use the compound of formula (I) in the manufacture of a medicament for use in the treatment or prevention of a disorder which is asthma, chronic obstructive pulmonary disease, allergic rhinitis, rheumatoid arthritis, multiple sclerosis, atopic dermatitis, psoriasis, inflammatory bowel disease.
According to still another embodiment the present invention covers a method for treating a subject afflicted with a pathological condition or disease susceptible to amelioration by inhibition of phosphodiesterase 4, which method comprises administering to the said subject an effective amount of a compound of formula (I), as hereinabove described. In a preferred embodiment the method is used for treating a subject afflicted with a pathological condition or disease which is asthma, chronic obstructive pulmonary disease, allergic rhinitis, rheumatoid arthritis, multiple sclerosis, atopic dermatitis, psoriasis, inflammatory bowel disease.

The compounds of the present invention may be prepared by methods such as those illustrated in the following scheme. Solvents, temperatures, pressures and other reaction conditions may readily be selected by one of ordinary skill in the art. Starting materials are commercially available or can be readily prepared by one of ordinary skill in the art using known methods. For all the schemes and compounds described below, R¹ and R² are as described for a compound of general formula (I).

Compounds of general formula (I) may be prepared following the synthetic scheme depicted in figure 1.

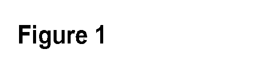

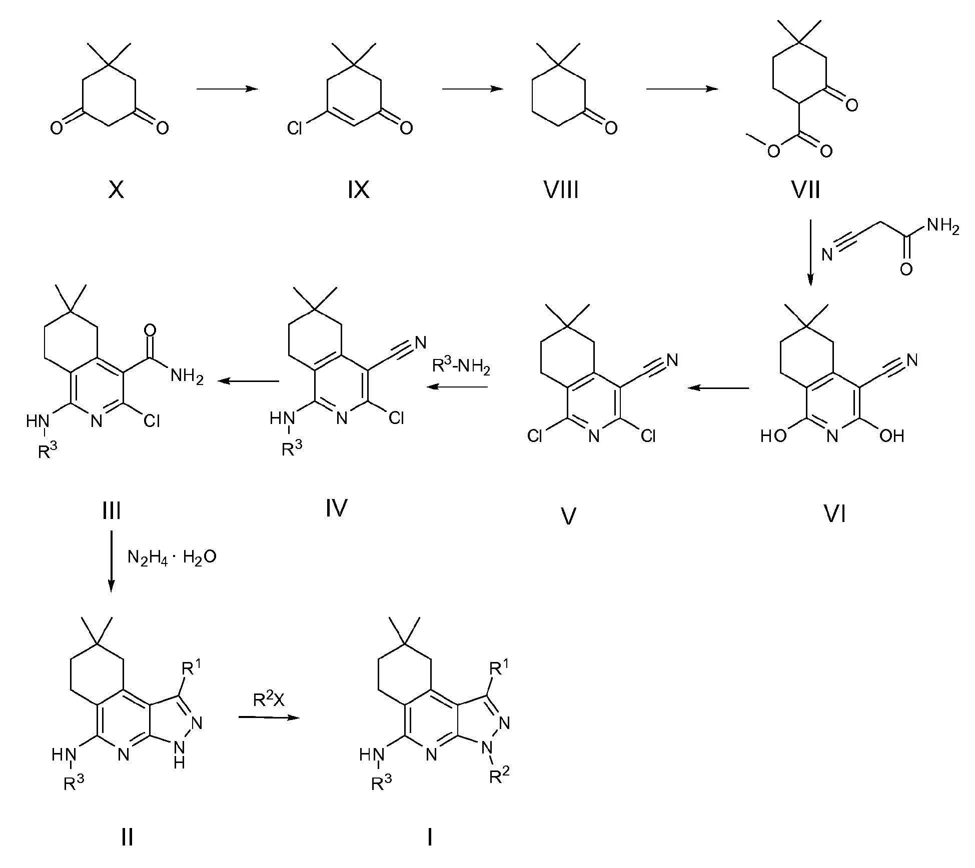

Intermediate **X**, which is commercially available, reacts with oxalyl chloride in the presence of DMF to yield 3-chloro-5,5-dimethylcyclohex-2-enone **IX,** according to the method described by R.E. Mewshaw at Tetrahedron Letters , 1989, 30(29), 3753-6 or by H. Ohmori et al. at the Journal of the Chemical Society, Chemical Communications, 1988, (13), 874-5.

Reaction of intermediate **IX** with hydrogen under pressure using a catalyst like palladium yields 3,3-dimethylcyclohexanone **VIII,** as described by F. Sondheimer and W. Saul at Canadian Journal of Chemistry, 1959, 37,1870-80 or by U. Ghatak et al. at the Journal of the American Chemical Society ,1957, 79, 4487-91.

Subsequent reaction of intermediate **VIII** with dimethylcarbonate in the presence of a base such as sodium hydride affords the 1,3-dicarbonyl derivative **VII,** according to D.L. Boger and M.D. Mullican at Journal of Organic Chemistry, 1985, 50(11), 1904-11.

The pyridine derivative **VI** may be prepared by cyclisation of intermediate **VII** with cyanoacetamide under refluxing conditions in the presence of a potassium hydroxide, as described by E. Wenkert et al at J.Am.Chem.Soc., 1965, 87, 5461. The same reference applies for the conversion of **VI** to the 1,6-dichloropyridine derivative **V** by reaction with phosphorous oxychloride without solvent at 150-170°C in a sealed tube.

Conversion of intermediate **V** to **IV** is carried out in a solvent, preferably a polar aprotic solvent, such as N,N-dimethylformamide, dioxane, acetone or tetrahydrofuran, in the presence of a strong base, such as sodium hydride or potassium tert-butoxide and at a temperature from 100°C to 150°C. In the R³-NH₂ reactant, R³ is defined as before.

The corresponding derivative of **III** may be prepared using concentrated sulfuric acid to give the desired final intermediate **III**, which is used in the next synthetic step without further purification. Cyclization of **III** with hydrazine in a solvent such as butanol under heating conditions yields intermediate **II**, as described for similar compounds by E.G. Paronikyan et al. at the Pharmaceutical Chemistry Journal (Translation of Khimiko-Farmatsevticheskii Zhurnal), 2001, 35(1), 8-10.

Compounds **I** may be prepared by reaction of **II** with R²X, where R² is defined as before and X is a leaving group, preferably a halogen atom (Br, CI or I), although leaving groups such as mesylate or tosylate are also suitable. The reaction is carried out in a solvent, preferably a polar aprotic solvent, such as N,N-dimethylformamide, dioxane, acetone or tetrahydrofuran, in the presence of a strong base, such as sodium hydride or potassium tert-butoxide and at a temperature ranging from room temperature to 100°C.

In the particular case wherein R¹ represents an amino group, compounds of general formula (Ia) may be prepared following the synthetic scheme depicted in figure 2.

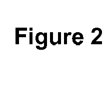

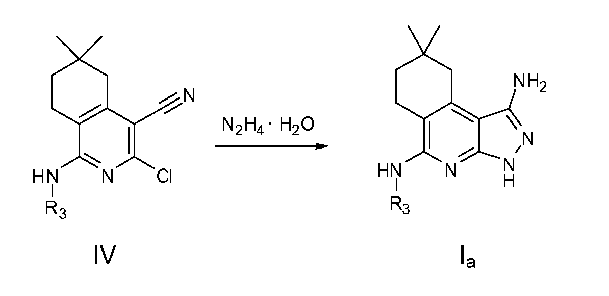

Compounds of formula **(Ia)** may be prepared by cyclization of intermediate **IV** with hydrazine in a solvent like butanol under heating conditions, following the same procedure as described for similar compounds by E.G. Paronikyan et al. at the Pharmaceutical Chemistry Journal (Translation of Khimiko-Farmatsevticheskii Zhurnal), 2001, 35(1), 8-10.

In the case of conversion of groups that are susceptible to chemical reaction under the conditions of the hereinbefore described processes or that are incompatible with said processes, conventional protecting groups may be used in accordance with standard practice, for example see T. W. Greene and P. G. M. Wuts in 'Protective Groups in Organic Chemistry', 3rd Edition, John Wiley & Sons (1999). In this case, deprotection may be produced in the last step in accordance with standard practice (see the above mentioned reference), yielding the final compounds of formula **I**.

The pharmaceutically acceptable salts of the compounds of the present invention represented by formula (V) may be acid addition salts or alkali addition salts. Examples of the acid addition salts include mineral acid addition salts such as, for example, hydrochloride, hydrobromide, hydroiodide, sulfate, nitrate, phosphate, and organic acid addition salts such as, for example, acetate, maleate, fumarate, citrate, oxalate, succinate, tartrate, malate, mandelate, methanesulfonate, and p-toluenesulfonate. Examples of the alkali addition salts include inorganic salts such as, for example sodium, potassium, calcium and ammonium salts and organic alkali salts such as, for example, ethylenediamine, ethanolamine, N,N-dialkylenethanolamine, triethanolamine and basic amino acid salts.

The compounds of the present invention represented by the above described formula (I) may include enantiomers depending on their asymmetry or diastereoisomers. The single isomers and mixtures of the isomers fall within the scope of the present invention.

The syntheses of the compounds of the invention and of the intermediates for use therein are illustrated by the following Examples (1-17) (including Preparation Examples (Preparations 1 to 12) which do not limit the scope of the invention in any way.

¹H Nuclear Magnetic Resonance Spectra were recorded on a Varian Gemini 200 spectrometer.

Low Resolution Mass Spectra (m/z) were recorded on a Micromass ZMD mass spectrometer using ESI ionization.

Melting points were recorded using a Perkin Elmer DSC-7 apparatus.

The chromatographic separations were obtained using a Waters 2690 system equipped with a Symmetry C18 (2.1 x 10 mm, 3.5 mM) column. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A): initially from 0% to 95% of B in 20 min, and then 4 min. with 95% of B. The reequilibration time between two injections was 5 min. The flow rate was 0.4 mL/min. The injection volume was 5 microliter. Diode array chromatograms were collected at 210 nM.

### Preparation Examples

### PREPARATION 1

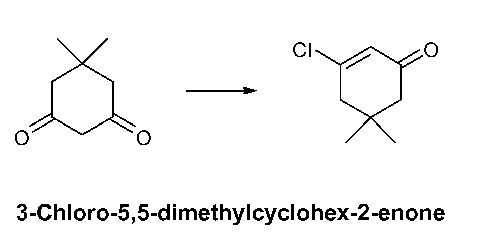

5,5-Dimethylcyclohexane-1,3-dione (20g, 0.14mol) is suspended in toluene (75ml). After the addition of five drops of DMF, oxalyl chloride is dropwise added (15.75ml, 0.18mol). This mixture is heated to 85°C for 2 h. After evaporation of the solvent at reduced pressure, the desired final compound is obtained as an oil (24g) and used at the following step without further purification.

¹H NMR (200 MHz, CHLOROFORM-d) δ ppm 1.09 (s, 6 H) 2.25 (s, 2 H) 2.56 (s, 2 H) 6.22 (s, 1 H)

### PREPARATION 2

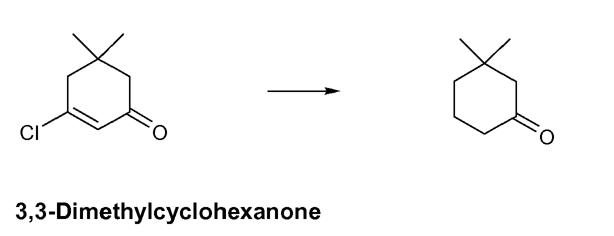

3-Chloro-5,5-dimethylcyclohex-2-enone (24g, 0.15mol. see Preparation 1) is suspended in pentane (200ml). Triethylamine (26ml, 0.19mol) and palladium (1.5g of Pd/C 10%, 0.01mol) are added. The reaction mixture is hydrogenated at 35 psi during 18 hours. After usual work-up, 11.8g of the final product is obtained after purification by distillation.

¹H NMR (200 MHz, CHLOROFORM-d) δ ppm 0.88 - 0.96 (m, 6 H) 1.42 - 1.59 (m, 2 H) 1.73 - 1.94 (m, 2 H) 2.10 (s, 2 H) 2.22 (t, *J*=7.03 Hz, 2 H)

### PREPARATION 3

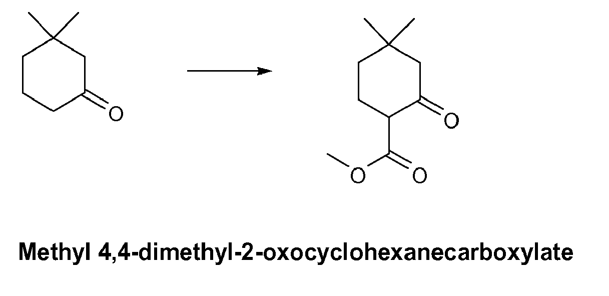

To a mixture of sodium hydride (7.7g, 0.19mol) in THF (200 ml), dimethylcarbonate (39.5 ml, 0.47mol) is added and heated to reflux. 3,3-Dimethylcyclohexanone (11.8g, 0.09mol, see Preparation 2) in THF (100 ml) is dropwise added and heated with stirring for 2h. After cooling until room temperature, the reaction mixture is poured on a saturated solution of ammonium chloride (300 ml) and extracted with ether. After usual work-up, 17.9g of the final compound are obtained as an oil.

¹H NMR (200 MHz, CHLOROFORM-d) δ ppm 0.87 - 1.00 (m, 6 H) 1.33 - 1.47 (m, 2 H) 1.57 - 1.75 (m, 1 H) 2.04 (s, 2 H) 2.15 - 2.30 (m, 2 H) 3.72 - 3.78 (m, 3 H)

### PREPARATION 4

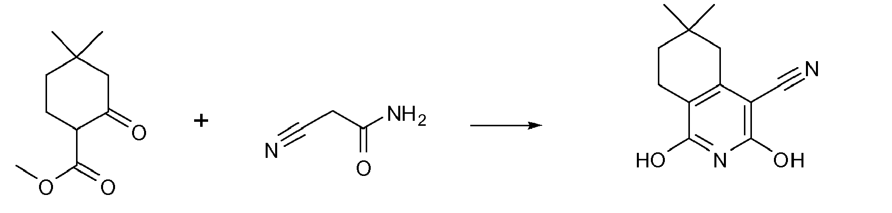

### 1,3-Dihydroxy-6,6-dimethyl-5,6,7,8-tetrahydroisoquinoline-4-carbonitrile

Methyl 4,4-dimethyl-2-oxocyclohexanecarboxylate (5.7g, 30.9mmol, see Preparation 3) is dissolved in methanol (25 ml) and cyanoacetamide (2.6g, 30.9mmol) is added. A solution of potassium hydroxide (1.8g, 32.4mmol) in methanol (7 ml) is dropwise added. The mixture is stirred at room temperature for 30 minutes and refluxed overnight. Once at room temperature, the solid formed is filtered and rinsed with methanol. This solid is suspended in water and acidified with concentrated chlorhydric acid. After stirring at 90°C for 3h, the solid is filtered and washed with water. 2.73g of the final compound are obtained.

¹H NMR (200 MHz, DMSO-*d*₆) δ ppm 0.93 (s, 6 H) 1.45 (t, *J*=6.44 Hz, 2 H) 2.28 - 2.41 (m, 4 H)

### PREPARATION 5

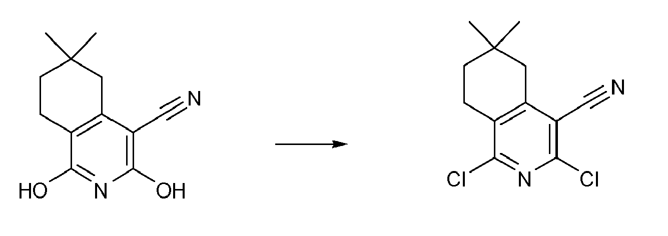

### 1,3-Dichloro-6,6-dimethyl-5,6,7,8-tetrahydroisoquinoline-4-carbonitrile

1,3-Dihydroxy-6,6-dimethyl-5,6,7,8-tetrahydroisoquinoline-4-carbonitrile (2.73g, 12.51mmol, see Preparation 4) and phosphorous oxychloride (8ml, 12.5mmol) are overnight heated in a sealed vessel at 170°C. Once at room temperature, this mixture is poured on 2N NaOH/ice, extracted with ethyl acetate, dried over magnesium sulfate and evaporated under reduced pressure. 1.72g of the final product is obtained as a solid.

¹H NMR (400 MHz, CHLOROFORM-d) δ ppm 1.03 (s, 6 H) 1.67 (t, *J*=6.85 Hz, 2 H) 2.74 (s, 2 H) 2.78 (t, *J*=6.65 Hz, 2 H)

### PREPARATION 6

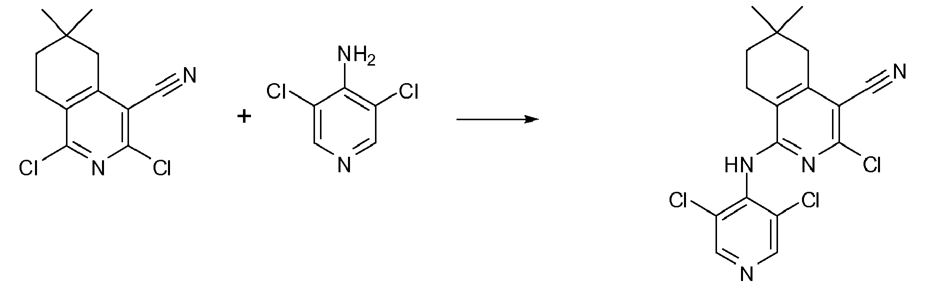

### 3-Chloro-1-(3,5-dichloropyridin-4-ylamino)-6,6-dimethyl-5,6,7,8-tetrahydroisoquinoline-4-carbonitrile

1,3-Dichloro-6,6-dimethyl-5,6,7,8-tetrahydroisoquinoline-4-carbonitrile (2.0g, 7.84 mmol, see Preparation 5), 3,5-dichloropyridine-4-amine (1.5g, 9.4 mmol), potassium *tert-*butoxide (1.76g, 15.7 mmol), Pd₂(dba)₃ (72 mg, 0.08 mmol) and 1,3-bis(2,6-diisopropylphenyl)imidazolium chloride (67 mg, 0.16 mmol) are suspended in dioxane (30 ml) and the system is purged with argon. After heating at 100°C for 2h, the reaction mixture is poured on water and extracted with ethyl acetate. The remaining aqueous phase is acidified with 2N HCI and extracted with ethyl acetate. After usual work-up, the crude residue is purified by flash chromatography eluting with dichloromethane/methanol 98:2 and 1.23g of the final product are isolated. Yield= 41%.

¹H NMR (200 MHz, CHLOROFORM-d) δ ppm 1.1 (s, 6 H) 1.7 (t, *J*=6.6 Hz, 2 H) 2.6 (t, *J*=6.6 Hz, 2 H) 2.7 (s, 2 H) 6.4 (s, 1 H) 8.5 (s, 2 H)

### PREPARATION 7

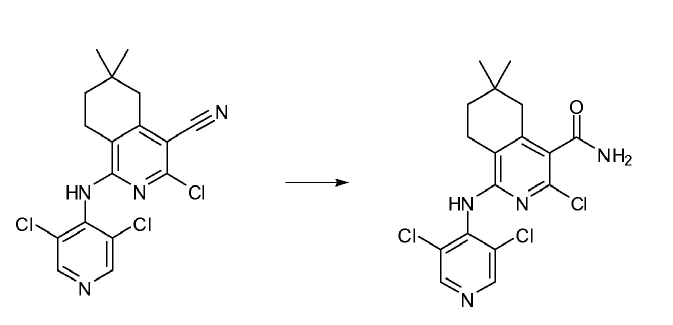

### 3-Chloro-1-(3,5-dichloropyridin-4-ylamino)-6,6-dimethyl-5,6,7,8-tetrahydroisoquinoline-4-carboxamide

3-Chloro-1-(3,5-dichloropyridin-4-ylamino)-6,6-dimethyl-5,6,7,8-tetrahydroisoquinoline-4-carbonitrile (100 mg, 0.26 mmol, see Preparation 6) is suspended in concentrated sulfuric acid (1 ml) and the mixture is heated at 60°C. Once the reaction is over, the mixture is poured on ice, basified with ammonia and extracted with chloroform. After usual work-up, 62 mg of the desired final product are obtained as a white solid, which is used at the next synthetic step without further purification.
HPLC/MS (15 min) retention time: 5.58
LRMS: *m*/*z* 399 (M⁺)

### PREPARATION 8

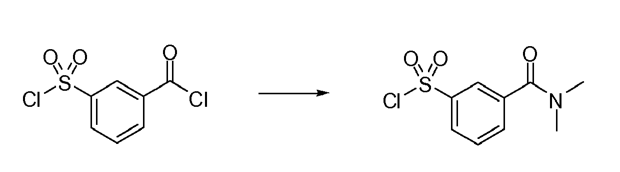

### 3-(Dimethylcarbamoyl)benzene-1-sulfonyl chloride

3-(Chlorosulfonyl)benzoyl chloride (5g, 20.9 mmol) in toluene (50 ml) is cooled to 0°C and a 2M solution of dimethylamine in THF (20.9 ml, 41.8 mmol) is dropwise added during 45 minutes. Once the addition is over, the reaction is stirred overnight at room temperature. The reaction mixture is poured on water, the organic phase is successively washed with water and brine, dried over magnesium sulfate, filtrate and the solvent is evaporated under reduced pressure. The residue is purified by flash chromatography eluting with hexane/AcOEt 7:3, isolating 2.8g of the final compound as an oil. Yield= 55%.

¹H NMR (200 MHz, CHLOROFORM-d) δ ppm 3.0 (s, 3 H) 3.2 (s, 3 H) 7.7 (m, 1 H) 7.8 (m, 1 H) 8.1 (m, 2 H)

### PREPARATION 9

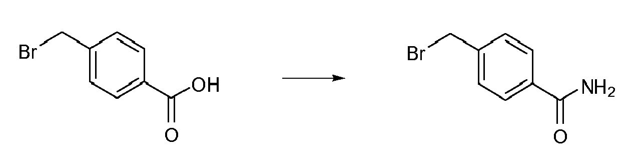

### 4-Bromomethylbenzamide

4-Bromomethylbenzoic acid (100mg, 0.47mmol) is suspended in ethyl acetate and thionyl chloride (51µl, 0.70mmol) is added. This mixture is heated at 75°C for 20 h. Then, it is cooled to 0°C and concentrated ammonia (56,4µl) is added and stirred during 20 min. Water is added and then it is filtered through Celite®. This aqueous phase is extracted with ethyl acetate, washed with 2N HCl, water and brine, dried over magnesium sulfate, filtered and the solvents evaporated under reduced pressure. 40mg of the final product are obtained as a solid.

¹H NMR (200 MHz, DMSO-*d*₆) δ ppm 4.68 - 4.98 (m, 2 H) 7.38 - 7.71 (m, 2 H) 7.78 - 8.16 (m, 2 H)

### PREPARATION 10

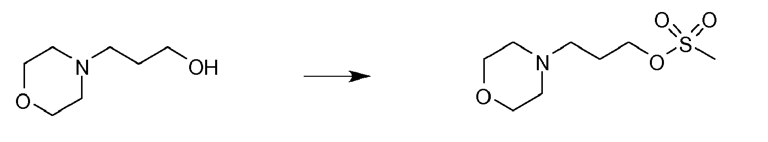

### 3-Morpholinopropyl methanesulfonate

3-Morpholinopropan-1-ol (0.5g, 3.44 mmol) and triethylamine (0.72 ml, 5.17 mmol) are dissolved in dichloromethane (10 ml). Mesityl chloride (0.40 ml, 5.17 mmol) in dichloromethane (5 ml) is dropwise added and stirred overnight at room temperature. After usual work-up, 0.7 g of the final product are obtained as an oil, which is used in the next synthetic step without further purification.

¹H NMR (200 MHz, CHLOROFORM-*d*) δ ppm 1.83 - 2.11 (m, 2 H) 2.35 - 2.59 (m, 4 H) 3.03 (s, 3 H) 3.47 - 3.67 (m, 2 H) 3.67 - 3.80 (m, 2 H) 4.17 - 4.44 (m, 4 H)

### PREPARATION 11

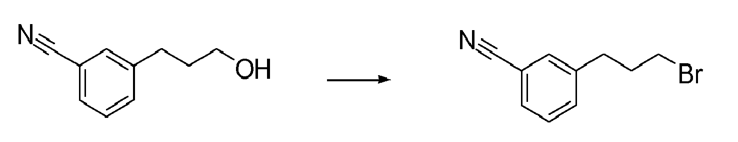

### 3-(3-Bromopropyl)benzonitrile

Triphenylphosphine (5.0g, 19.1mmol) in acetonitrile (7 ml) is cooled at 0°C and bromine (1ml, 19.1mmol) dissolved in acetonitrile (14 ml) is dropwise added. The ice-bath is removed and, once at room temperature, 3-(3-hydroxypropyl)benzonitrile (2.8g, 17.4 mmol) dissolved in acetonitrile (7 ml) is dropwise added. When the addition is over, the reaction mixture is heated to 50°C for 18 h. The solvent is evaporated, toluene is added and then it is removed under reduced pressure. The residue is suspended in toluene, filtered and the liquid phase is evaporated to dryness. The oily residue is purified through a path of silica eluting with hexane/AcOEt (9:1) to get 2.23g of the final compound.

¹H NMR (200 MHz, CHLOROFORM-d) δ ppm 2.00 - 2.32 (m, 2 H) 2.84 (t, *J*=7.42 Hz, 2 H) 3.39 (t, *J*=5.86 Hz, 2 H) 7.29 - 7.70 (m, 4 H)

### PREPARATION 12

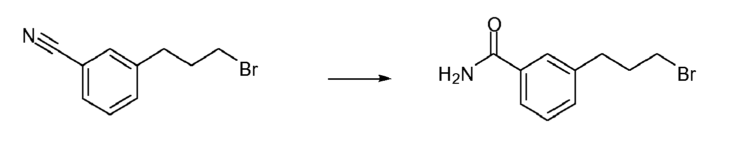

### 3-(3-Bromopropyl)benzamide

3-(3-Bromopropyl)benzonitrile (2g, 8.92mmol) and potassium bicarbonate (0.3g, 3 mmol) are dissolved in methanol (20 ml) and then hydrogen peroxide (2g, 17.8mmol) is added. After 45 minutes of stirring at room temperature, potassium carbonate (15g, 108.5mmol) in methanol (20 ml) is added and stirred overnight at room temperature. 40 ml of water are added and then this reaction mixture is left at 5°C for a while. A semi-solid is filtered and purified by flash chromatography eluting with hexane/AcOEt 2:1 to isolate 0.56g of the final product as a solid.

¹H NMR (200 MHz, DMSO-*d*₆) δ ppm 1.96 - 2.24 (m, 2 H) 2.67 - 2.87 (m, 2 H) 3.52 (t, *J*=6.64 Hz, 2 H) 7.20 - 7.43 (m, 3 H) 7.63 - 7.76 (m, 2 H) 7.93 (s, 1 H)

### EXAMPLE 1

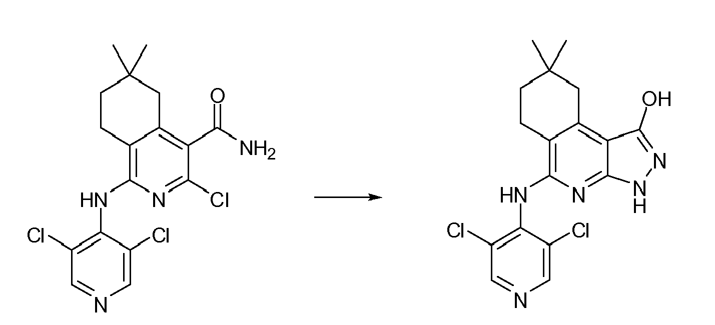

### 5-(3,5-Dichloropyridin-4-ylamino)-8,8-dimethyl-6,7,8,9-tetrahydro-3H-pyrazolo[3,4-c]isoquinolin-1-ol

3-Chloro-1-(3,5-dichloropyridin-4-ylamino)-6,6-dimethyl-5,6,7,8-tetrahydroisoquinoline-4-carboxamide (62 mg, 0.16 mmol, see Preparation 7) is dissolved in butanol (5 ml) and hydrazine monohydrate (152 µl, 3.10 mmol) is added. After overnight heating to reflux, additional hydrazine monohydrate is added (0.5 ml) and heated overnight again. After evaporation of the solvents, the residue is dissolved in 2N NaOH and extracted with chloroform. The aqueous phase is neutralized with 2N HCl and extracted with chloroform. After usual work-up, the residue is purified by flash chromatography eluting with DCM/MeOH 95:5 to yield 13 mg of the final product as a white solid.

¹H NMR (200 MHz, DMSO-*d*₆) δ ppm 1.00 (s, 6 H) 1.64 (s, 2 H) 2.50 (s, 2 H) 2.83 (s, 2 H) 8.27 (s, 1 H) 8.65 (s, 2 H)

### EXAMPLE 2

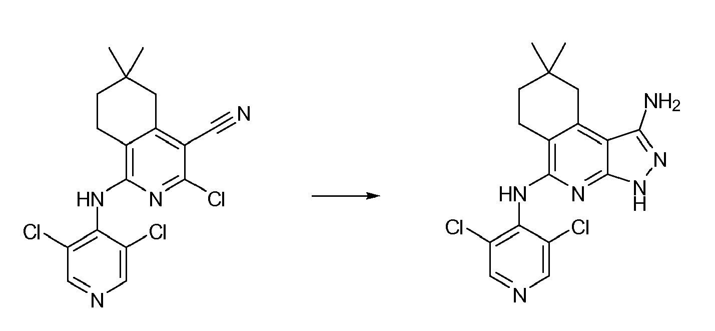

### N⁵-(3,5-Dichloropyridin-4-yl)-8,8-dimethyl-6,7,8,9-tetrahydro-3H-pyrazolo[3,4-c]isoquinoline-1,5-diamine

3-Chloro-1-(3,5-dichloropyridin-4-ylamino)-6,6-dimethyl-5,6,7,8-tetrahydroisoquinoline-4-carbonitrile (100 mg, 0.26 mmol, see Preparation 6) is dissolved in ethanol (1 ml) and hydrazine monohydrate (255 µl, 5.23mmol) is added. After overnight heating to reflux, the solvent is evaporated under reduced pressure and the residue is suspended in water, filtered, washed with water and ether and recrystallized from ethanol. 26 mg of the final product are obtained.

¹H NMR (200 MHz, CHLOROFORM-d) δ ppm 1.1 (s, 6 H) 1.8 (t, *J*=6.8 Hz, 2 H) 2.7 (m, 2 H) 2.9 (s, 2 H) 4.0 (s, 2 H) 6.4 (s, 1 H) 8.5 (s, 2 H) 8.9 (s, 1 H)

### EXAMPLE 3

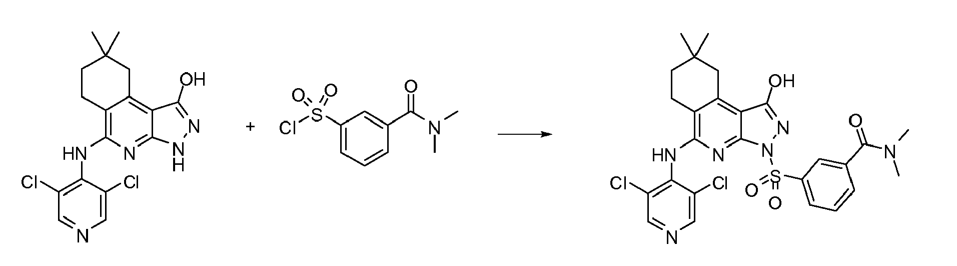

### 3-(5-(3,5-Dichloropyridin-4-ylamino)-1-hydroxy-8,8-dimethyl-6,7,8,9-tetrahydro-3H-pyrazolo[3,4-c]isoquinolin-3-ylsulfonyl)-N, N-dimethylbenzamide

5-(3,5-Dichloropyridin-4-ylamino)-8,8-dimethyl-6,7,8,9-tetrahydro-3H-pyrazolo[3,4-c]isoquinolin-l-ol (50 mg, 0.13 mmol, see Example 1) is dissolved in chloroform (2 ml). Triethylamine (20 µl, 0.14 mmol) and 3-(dimethylcarbamoyl)benzene-1-sulfonyl chloride (36 mg, 0.15 mmol, see Preparation 8) are added. The reaction mixture is stirred 3h at room temperature. After usual work-up, the residue is purified by flash chromatography eluting with hexane/AcOEt 3:7. 10 mg of the final product are obtained.
HPLC/MS (15 min) retention time: 8.67 min
LRMS: *m*/*z* 589 (M⁺)

### EXAMPLE 4

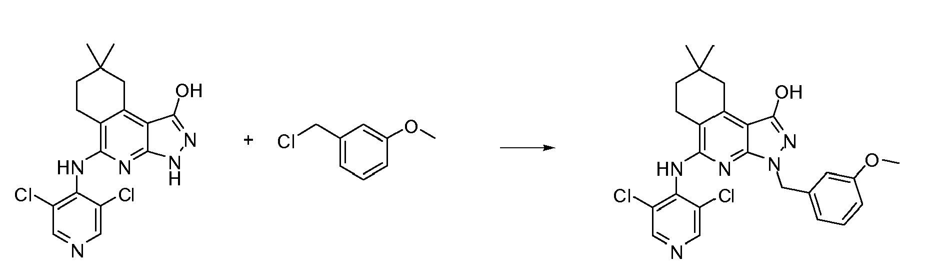

### 5-(3,5-Dichloropyridin-4-ylamino)-3-(3-methoxybenzyl)-8,8-dimethyl-6,7,8,9-tetrahydro-3H-pyrazolo[3,4-c]isoquinolin-1-ol

5-(3,5-Dichloropyridin-4-ylamino)-8,8-dimethyl-6,7,8,9-tetrahydro-3H-pyrazolo[3,4-c]isoquinolin-1-ol (50 mg, 0.13 mmol, see Example 1) is dissolved in DMF (2 ml) and sodium hydride (60% in hexanes, 6.5 mg, 0.18 mmol) is added. This mixture is stirred under nitrogen at room temperature for 10 minutes. Then, 3-methoxybenzyl chloride (25mg, 0.16 mmol) dissolved in DMF (1 ml) is added and the mixture is stirred overnight at room temperature. After diluting the reaction mixture with water, a solid precipitates, which is filtered and recrystallized from ethanol to yield 4mg of the final compound.
HPLC/MS (15 min) retention time: 9.99 min
LRMS: *m*/*z* 498 (M⁺)

### EXAMPLE 5

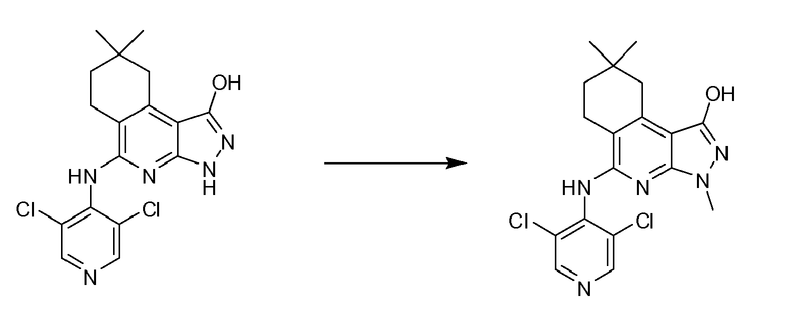

### 5-(3,5-Dichloropyridin-4-ylamino)-3,8,8-trimethyl-6,7,8,9-tetrahydro-3H-pyrazolo[3,4-c]isoquinolin-1-ol

5-(3,5-Dichloropyridin-4-ylamino)-8,8-dimethyl-6,7,8,9-tetrahydro-3H-pyrazolo[3,4-c]isoquinolin-1-ol (50 mg, 0.13 mmol, see Example 1) is dissolved in DMF (1.5 ml) and sodium hydride (60% in hexanes, 6.5 mg, 0.16 mmol) is added under nitrogen. After 10 minutes of stirring, methyl iodide (10 µl, 0.16 mmol) in DMF (0.5 ml) is added and the mixture is stirred overnight at room temperature. After usual work-up, the reaction mixture is purified by chromatography to yield 14 mg of the final product.
HPLC/MS (15 min) retention time: 7.93
LRMS: *m*/*z* 392 (M⁺)

### EXAMPLE 6

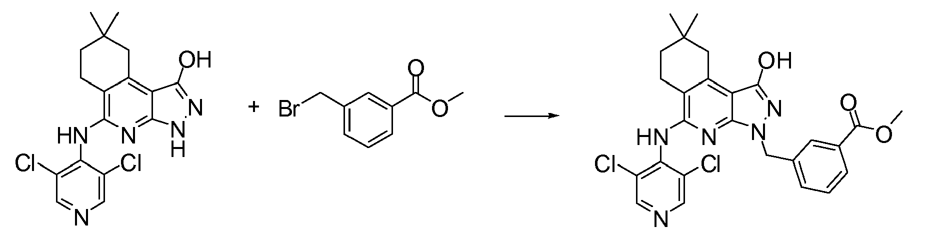

### Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate

Reaction of the title compound of Example 1 (100 mg, 0.26 mmol) with methyl 3-(bromomethyl)benzoate (73 mg, 0.32 mmol) according to the method described in Example 4 gave 97 mg (63%) of the title compound.
HPLC/MS (15 min) retention time 8.87 min.
LRMS: *m*/*z* 595 (M+)

### EXAMPLE 7

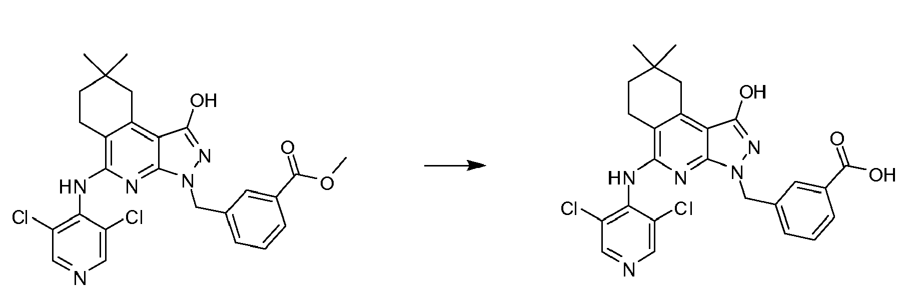

### 3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid

Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate (80 mg, 0.15 mmol) is dissolved in THF (2 ml) and lithium hydroxide (20mg, 0.48 mmol) in water (0.5 ml) is added. The reaction mixture is stirred overnight at room temperature. The solvent is evaporated under reduced pressure and the residue is suspended in water. After acidification with 2N HCl until pH=3, a solid precipitates, which is filtered and washed with water and ether. 51 mg of the final product are obtained. Yield= 65%.

¹H NMR (200 MHz, DMSO-*d*₆) δ ppm 1.00 (s, 6 H) 1.65 (s, 2 H) 2.65 (s, 2 H) 2.79 (s, 2 H) 4.94 (s, 2 H) 7.36 (d, *J*=7.03 Hz, 2 H) 7.69 (s, 1 H) 7.79 (d, *J*=7.81 Hz, 1 H) 8.30 (s, 1 H) 8.65 (s, 2 H)

### EXAMPLE 8

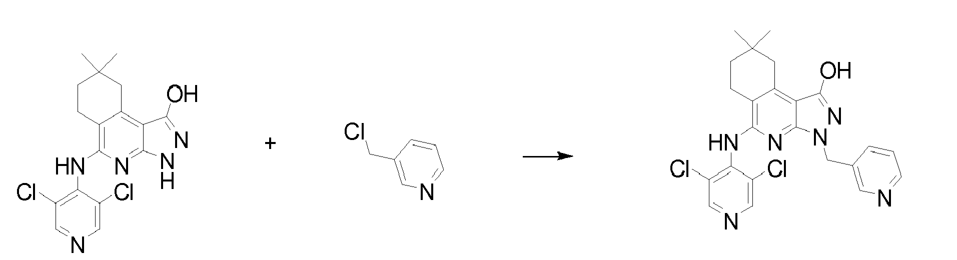

### 5-(3,5-Dichloropyridin-4-ylamino)-8,8-dimethyl-3-(pyridin-3-ylmethyl)-6,7,8,9-tetrahydro-3H-pyrazolo[3,4-c]isoquinolin-1-ol

Reaction of the title compound of Example 1 (50 mg, 0.13 mmol) with 3-chloromethylpyridine (21 mg, 0.16 mmol) according to the method described in Example 4 gave 6 mg (10%) of the title compound.
HPLC/MS (15 min) retention time 7.35 min.
LRMS: *m*/*z* 469 (M+)

### EXAMPLE 9

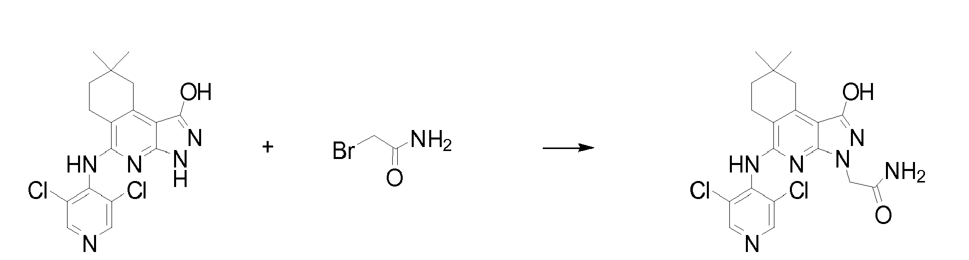

### 2-(5-(3,5-Dichloropyridin-4-ylamino)-1-hydroxy-8,8-dimethyl-6,7,8,9-tetrahydro-3H-pyrazolo[3,4-c]isoquinolin-3-yl)acetamide

Reaction of the title compound of Example 1 (50 mg, 0.13 mmol) with 3-bromoacetamide (20 mg, 0.15 mmol) according to the method described in Example 4 gave 8 mg (14%) of the title compound.
HPLC/MS (15 min) retention time 7.28 min.
LRMS: *m*/*z* 435 (M+)

### EXAMPLE 10

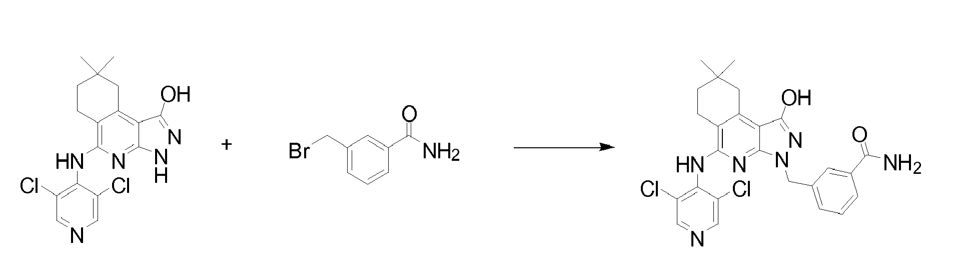

### 3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide

Reaction of the title compound of Example 1 (100 mg, 0.26 mmol) with 3-(bromomethyl)benzamide (73 mg, 0.32 mmol) according to the method described in Example 4 gave 8 mg (14%) of the title compound.

¹H NMR (200 MHz, CHLOROFORM-d) δ ppm 1.07 (s, 6 H) 1.21 - 1.31 (m, 2 H) 1.63 - 1.84 (m, 2 H) 2.62 - 2.77 (m, 2 H) 3.00 (s, 2 H) 4.97 (s, 2 H) 6.52 (s, 1 H) 7.35 - 7.47 (m, 2 H) 7.69 (s, 1 H) 7.93 (s, 1 H) 8.56 (s, 2 H) 8.97 (s, 1 H)
HPLC/MS (15 min) retention time 6.27 min.
LRMS: *m*/*z* 511 (M+)

### EXAMPLE 11

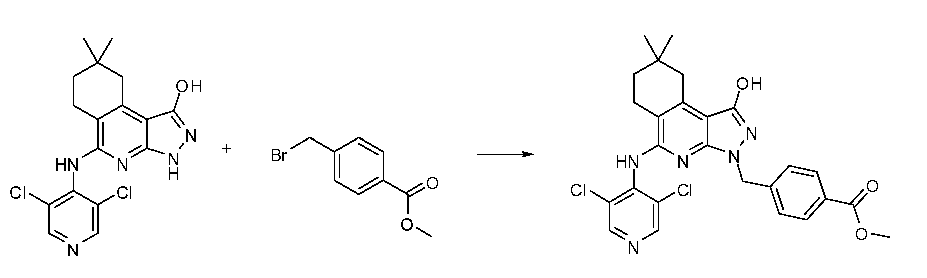

### Methyl 4-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate

Reaction of the title compound of Example 1 (100 mg, 0.26 mmol) with methyl 4-(bromomethyl)benzoate (73 mg, 0.32 mmol) according to the method described in Example 4 gave 41 mg (29%) of the title compound.

¹H NMR (200 MHz, DMSO-*d*₆) δ ppm 1.00 (s, 6 H) 1.65 (m, 2 H) 2.65 (m, 2 H) 2.79 (s, 2 H) 3.85 (s, 3H) 4.94 (s, 2 H) 7.20 (d, *J*=7.03 Hz, 2 H) 7.85 (d, *J*=7.81 Hz, 1 H) 8.30 (bs, 1 H) 8.65 (s, 2 H) 10.6 (bs, 1H).
HPLC/MS (9 min) retention time 6.88 min.
LRMS: *m*/*z* 526 (M+)

### EXAMPLE 12

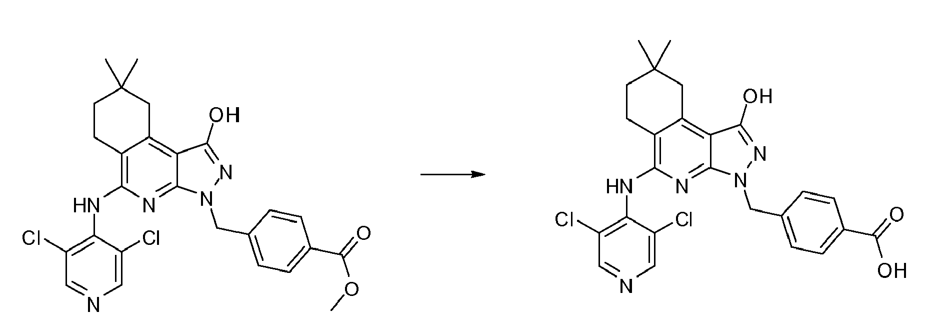

### 4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid

Reaction of the title compound of Example 11 (40 mg, 0.08 mmol) with lithium hydroxide (9.6 mg, 0.23 mmol) according to the method described in Example 7 gave 38 mg (98%) of the title compound.

¹H NMR (400 MHz, DMSO-*d*₆) δ ppm 1.00 (s, 6 H) 1.64 (t, *J*=6.26 Hz, 2 H) 2.64 (t, *J*=6.26 Hz, 2 H) 2.79 (s, 2 H) 4.94 (s, 2 H) 7.15 (d, *J*=8.22 Hz, 2 H) 7.81 (d, *J*=8.22 Hz, 2 H) 8.33 (s, 1 H) 8.66 (s, 2 H)
HPLC/MS (15 min) retention time 8.26 min.
LRMS: *m*/*z* 512 (M+)

### EXAMPLE 13

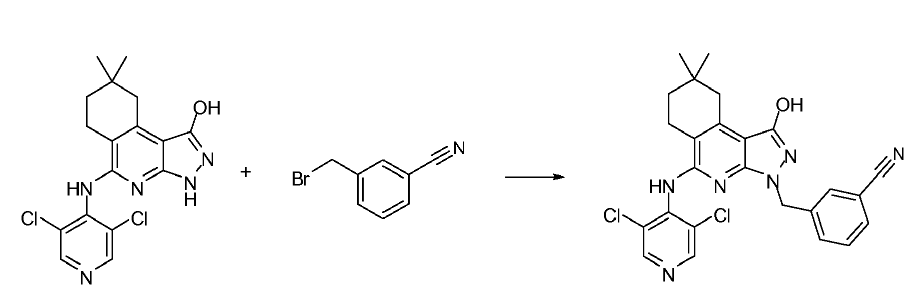

### 3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzonitrile

Reaction of the title compound of Example 1 (50 mg, 0.13 mmol) with 3-(bromomethyl)benzonitrile (31 mg, 0.16 mmol) according to the method described in Example 4 gave 17 mg (26%) of the title compound.

¹H NMR (200 MHz, CHLOROFORM-*d*) δ ppm 0.92 - 1.19 (m, 6 H) 1.60 - 1.85 (m, 2 H) 2.58 - 2.79 (m, 2 H) 2.90 - 3.03 (m, 2 H) 5.08 (s, 2 H) 6.47 (s, 2 H) 7.42 - 7.65 (m, 4 H) 8.56 (s, 2 H)

### EXAMPLE 14

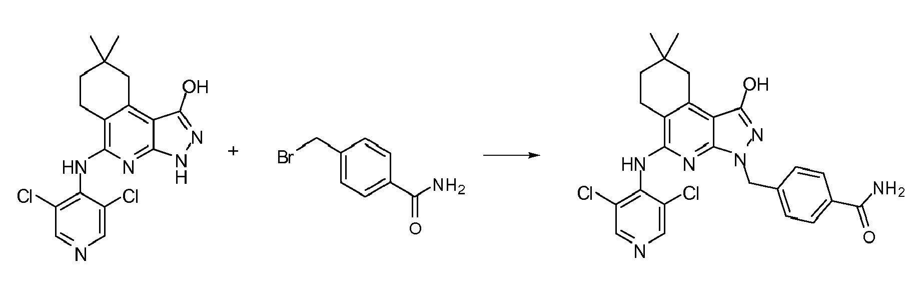

### 4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide

Reaction of the title compound of Example 1 (50 mg, 0.13 mmol) with 4-(bromomethyl)benzamide (34 mg, 0.16 mmol, see Preparation 9) according to the method described in Example 4 gave 10 mg (14%) of the title compound.

¹H NMR (200 MHz, CHLOROFORM-d) δ ppm 1.05 (s, 6 H) 1.21 - 1.31 (m, 2 H) 1.63 - 1.84 (m, 2 H) 2.62 - 2.77 (m, 2 H) 3.00 (s, 2 H) 4.95 (s, 2 H) 6.52 (s, 1 H) 7.15 (d, *J*=8.22 Hz, 2 H) 7.81 (d, *J*=8.22 Hz, 2 H) 8.56 (s, 2 H) 8.97 (s, 1 H)
HPLC/MS (9 min) retention time 6.19 min.
LRMS: *m*/*z* 511 (M+)

### EXAMPLE 15

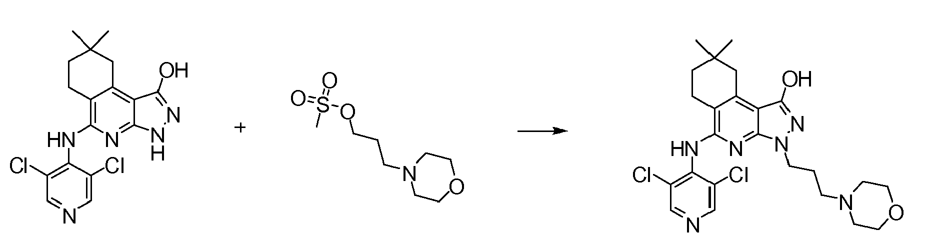

### 5-(3,5-Dichloropyridin-4-ylamino)-8,8-dimethyl-3-(3-morpholinopropyl)-6,7,8,9-tetrahydro-3H-pyrazolo[3,4-c]isoquinolin-1-ol

Reaction of the title compound of Example 1 (88 mg, 0.23 mmol) with 3-morpholinopropyl methanesulfonate (62 mg, 0.28 mmol, see Preparation 10) according to the method described in Example 4 gave 22 mg (26%) of the title compound.

¹H NMR (200 MHz, CHLOROFORM-d) δ ppm 1.08 (s, 6 H) 1.25 (m, 2 H) 1.75 (t, 2 H) 2.00 (m, 2 H) 2.50 - 2.70 (m, 8 H) 2.95 (s, 2 H) 3.8 - 3.9 (m, 4 H) 6.47 (s, 1 H) 8.50 (s, 2 H)
HPLC/MS (30 min) retention time 9.36 min.
LRMS: *m*/*z* 505 (M+)

### EXAMPLE 16

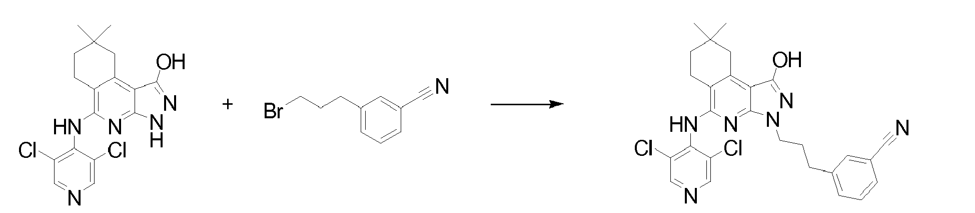

### 3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzonitrile

Reaction of the title compound of Example 1 (70 mg, 0.19 mmol) with 3-(3-bromopropyl)benzonitrile (50 mg, 0.22 mmol, see Preparation 11) according to the method described in Example 4 gave 42 mg (44%) of the title compound.

¹H NMR (200 MHz, CHLOROFORM-d) δ ppm 1.08 (s, 6 H) 1.75 (s, 2 H) 2.11 (s, 2 H) 2.42 - 2.77 (m, 4 H) 2.95 (s, 2 H) 3.79 - 4.12 (m, 2 H) 6.47 (s, 1 H) 7.18 - 7.54 (m, 4 H) 8.50 (s, 2 H)

### EXAMPLE 17

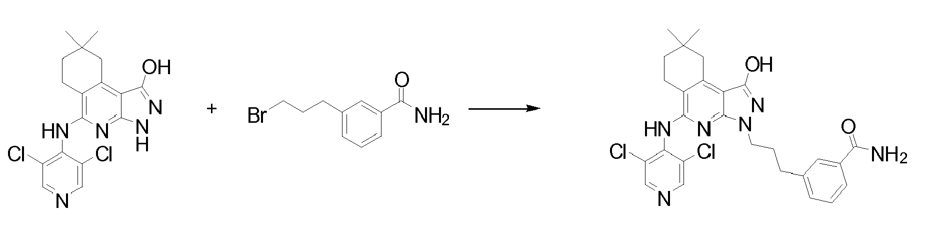

### 3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzamide

Reaction of the title compound of Example 1 (70 mg, 0.19 mmol) with 3-(3-bromopropyl)benzamide (54 mg, 0.22 mmol, see Preparation 12) according to the method described in Example 4 gave 55 mg (55%) of the title compound.

¹H NMR (200 MHz, CHLOROFORM-d) δ ppm 1.06 (s, 6 H) 1.56 - 1.80 (m, 2 H) 1.93 - 2.19 (m, 2 H) 2.38 - 2.76 (m, 4 H) 2.98 (s, 3 H) 3.88 (t, *J*=6.64 Hz, 2 H) 6.59 (s, 1 H) 7.15 - 7.43 (m, 4 H) 7.55 - 7.71 (m, 2 H) 8.47 (s, 2 H)

### PHARMACOLOGICAL ACTIVITY

### PDE4 Assay Procedure

Measurement of Phosphodiesterase Activity was done using a [3H] cAMP Scintillation Proximity Assay (SPA) (GE Healthcare). Compounds to be tested were dissolved in DMSO at a stock concentration of 1 mM and serial dilutions were prepared in 50% DMSO to determine IC50s.
The reactions were conducted in 96-well plates (Corning, Ref.3604) at room temperature, in 0.1 ml of reaction buffer containing (final concentrations): 50 mM Tris-HCl, pH 7.5, 8.3 mM MgCl₂, 1.7 mM EGTA, 30 nM [3H] cAMP (approximately 150000 dpm/well) with or without the inhibitors. The reaction was initiated by adding yeast extract of recombinant PDE4 enzyme.
Plates were shaken for 1 hr at room temperature and the incubation was terminated by adding 50 µl (0.5 mg /well) of SPA yttrium silicate beads (RPNQ 0150; GE Healthcare) in the presence of zinc sulfate. Plates were stored overnight in the dark and read on a TRILUX microtiter plate reader (Perkin Elmer).

The results are shown in Table 1.

| **Examples** | **IC₅₀ (nM)** |
|---|---|
| 1 | 3.90 |
| 2 | 62.00 |
| 3 | 16.80 |
| 4 | 11.00 |
| 5 | 17.10 |
| 6 | 2.00 |
| 7 | 3.10 |
| 8 | 7.40 |
| 9 | 18.8 |
| 10 | 2.30 |
| 12 | 2.40 |
| 13 | 2.35 |
| 14 | 5.17 |
| 15 | 6.14 |
| 16 | 15.50 |
| 17 | 2.10 |

It can be seen from Table 1 that the compounds of formula (I) are very potent inhibitors of phosphodiesterase 4 (PDE 4). The compounds are also capable of blocking the production of some pro-inflammatory cytokines such as, for example, TNFα.

Thus, they can be used in the treatment of allergic, inflammatory and immunological diseases, as well as those diseases or conditions where the blockade of pro-inflammatory cytokines or the selective inhibition of PDE 4 could be of benefit. These disease states include asthma, chronic obstructive pulmonary disease, allergic rhinitis, rheumatoid arthritis, osteoarthritis, osteoporosis, bone-formation disorders, glomerulonephritis, multiple sclerosis, ankylosing spondylitis, Graves ophtalmopathy, myasthenia gravis, diabetes insipidus, graft rejection, gastrointestinal disorders such as irritable bowel disease, ulcerative colitis or Crohn disease, septic shock, adult distress respiratory syndrome, and skin diseases such as atopic dermatitis, contact dermatitis, acute dermatomyositis and psoriasis. They can also be used as improvers of cerebrovascular function as well as in the treatment of other CNS related diseases such as dementia, Alzheimer's disease, depression, and as nootropic agents.

Like other PDE4 inhibitors (see references above) the compounds of the invention can also be used for blocking, after preventive and/or curative treatment, the erosive and ulcerogenic effects induced by a variety of etiological agents, such as antiinflammatory drugs (steroidal or non-steroidal antiinflammatory agents), stress, ammonia, ethanol and concentrated acids.

They can be used alone or in combination with antacids and/or antisecretory drugs in the preventive and/or curative treatment of gastrointestinal pathologies like drug-induced ulcers, peptic ulcers, H. Pylori-related ulcers, esophagitis and gastro-esophageal reflux disease.

They can also be used in the treatment of pathological situations where damage to the cells or tissues is produced through conditions like anoxia or the production of an excess of free radicals. Examples of such beneficial effects are the protection of cardiac tissue after coronary artery occlusion or the prolongation of cell and tissue viability when the compounds of the invention are added to preserving solutions intended for storage of transplant organs or fluids such as blood or sperm. They are also of benefit on tissue repair and wound healing.

The compounds of the present invention can also be used in combination with other drugs known to be effective in the treatment of these diseases for example (a) β2-adrenergic agonists, (b) anti-cholinergics, (c) anti-allergic agents, (d) ant-inflammatory agents, (e) immunosuppressants, and (f) anti-infectives; for simultaneous, separate or sequential use in the treatment of the human or animal body.

Accordingly, another embodiment of the invention is the use of the compounds of formula (I) in the manufacture of a medicament for treatment or prevention of pathological conditions, diseases and disorders known to be susceptible of amelioration by inhibition of PDE4, as well as a method for treating a subject afflicted with a pathological condition or disease susceptible to amelioration by inhibition of PDE4, which comprises administering to said subject an effective amount of a compound of formula (I).

The present invention also provides pharmaceutical compositions which comprise, as an active ingredient, at least a tetrahydropyrazolo[3,4-c]isoquinolin-5-amine derivative of formula (I) or a pharmaceutically acceptable salt thereof in association with a pharmaceutically acceptable excipient such as a carrier or diluent. The active ingredient may comprise 0.001 % to 99% by weight, preferably 0.01 % to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

The pharmaceutically acceptable excipients which are admixed with the active compound or salts of such compound, to form the compositions of this invention are well-known per se and the actual excipients used depend inter alia on the intended method of administering the compositions.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 0.01-3000 mg, more preferably 0.5-1000 mg of active ingredient or the equivalent amount of a pharmaceutically acceptable salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuiar® (formerly known as Novolizer SD2FL), which is described the following patent applications Nos: WO97/000703, WO03/000325 and WO03/061742.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even stricter.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. WO 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient(s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetrafluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient-free or may optionally contain additional formulation excipients well known in the art such as surfactants like oleic acid or lecithin and cosolvents like ethanol. Pressurised formulations will generally be retained in a canister (eg. an aluminium canister) closed with a valve (eg. a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg. by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate. Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

The compounds, salts and/or pharmaceutical compositions according to the invention may also be used in combination with another therapeutically active agent, for example a β2-adrenoreceptor agonist, an anti-cholinergic agent, an anti-allergic, an anti-inflammatory agent, an immunosuppressant, or an anti-infective agent.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day, most preferably once a day.

Examples of suitable β2-agonists that can be combined with PDE4 inhibitors are terbutaline sulphate, eformoterol fumarate, formoterol fumarate, bambuterol, procaterol hydrochloride, sibenadet hydrochloride, mabuterol hydrochloride, albuterol sulphate, salbutamol sulphate, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, Bedoradrine sulphate, Indacaterol, Trantinterol hydrochloride, AZD-3199, GSK-159802; GSK-597901, GSK-678007, GSK-642444;GSK-961081; AR-C98955AA, Milveterol hydrochloride, BI-1744-CL, and compounds described in international patent applications Nos. WO2007/124898, W02006/122788A1, WO2008/046598 and WO2008095720

Examples of suitable corticosteroids and glucocorticoids that can be combined with PDE4 inhibitors are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable M3 antagonists (anticholinergics) that can be combined with PDE4 inhibitors are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-allergic agents that can be combined with PDE4 inhibitors are anti-histamines (e.g. Methapyrilene, Mequitazine,Azelastine hydrochloride,Acrivastine, Emedastine difumarate; Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride,Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1004723D, GSK-835726, SUN-1334H), CRTH antagonists (e.g. Ramatroban, AMG-009, OC-000459), or mast cell stabilizers (e.g. nedocromil, pemirolast potassium, chromoglycate sodium, suplatast tosilate)

Other possible combinations include, for example, a combination comprising a PDE4 inhibitor with another anti-inflammatory agent such as a non-steroidal anti-inflammatory drug (NSAID) such as a leukotriene antagonist (e.g. Ibudilast, Pranlukast hydrate, Zafirlukast, Montelukast, Tipelukast), a FLAP inhibitor, a lipoxygenase inhibitor, a cyclooxygenase inhibitor (e.g. diclofenac, ibuprofen, celecoxib); an elastase inhibitor, a Syk kinase inhibitor or another PDE inhibitor (e.g. theophylline).

Examples for suitable immunosupressants that can be combined with PDE4 inhibitors are picremolimus, tacromilus, cyclosporine A, leflunomide, methotrexate, anti-TNF agents and compounds described in International patent applications Nos. PCT/EP2008/006573 and WO2008/077639.

Examples for suitable anti-infectives that can be combined with PDE4 inhibitors are mupiricin, retapamulin, clotrimazole, ketoconazole and terbinafine.

Particularly preferred pharmaceutical composition according to the invention comprise a salt of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, compounds described in International patent applications Nos. PCT/EP2008/006573 and WO2008/077639, methapyrilene, cetirizine, loratadine, ebastine, desloratadine, fexofenadine, azelastine, levocabastine, olopatadine, montelukast, picremolimus, tacromilus, mupiricin, retapamulin, clotrimazole, ketoconazole, terbinafine.

Thus, in one aspect of the invention, the composition comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the composition comprises a compound of formula (I) and an anticholinergic agent. Particularly preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. The composition may further comprise a corticosteroid selected from the group consisting of mometasone furoate, ciclesonide, budesonide, fluticasone furoate and fluticasone propionate.

In a still other aspect of the invention, the composition comprises a compound of formula (I) and a suitable β2-agonist. Particularly preferred β2-agonists are those selected from the group consisting of formoterol, salmeterol, indacaterol, carmoterol and compound described in International patent applications Nos. WO2007/124898, W02006/122788A1, WO2008/046598 and WO2008095720. The composition may further comprise a corticosteroid selected from the group consisting of mometasone furoate, ciclesonide, budesonide, fluticasone furoate, and fluticasone propionate. In addition to the salt of the invention and to the β2-agonist, the composition may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicycio[2.2.2] octane salts and 1-(2-Phenylethyl)-3-(9H-xanthen-9-ylcarbonyloxy)-1-azoniabicyclo[2.2.2] octane salts.

The compounds of formula (I) and the combinations of the invention may be used in the treatment of respiratory, skin and inflammatory diseases, wherein the use of a PDE4 inhibitor is expected to have a beneficial effect, for example asthma, chronic obstructive pulmonary disease, allergic rhinitis, rheumatoid arthritis, multiple sclerosis, atopic dermatitis, psoriasis, inflammatory bowel disease.

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies the preferred application form, especially in the therapy of obstructive lung diseases or for the treatment of asthma.

Additional suitable carriers for formulations of the active salts of the present invention can be found in Remington: The Science and Practice of Pharmacy, 20th Edition, Lippincott Williams & Wilkins, Philadelphia, Pa., 2000. The following non-limiting examples illustrate representative pharmaceutical compositions of the invention.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I) or a tautomer thereof: 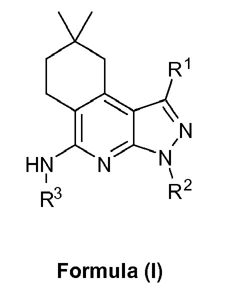 wherein:
• R¹ is selected from the group consisting of a hydroxy group and an amino group,
• R² is selected from the group consisting of a hydrogen atom, a linear or branched C₁₋₄ alkyl group, -(CH₂)₍₀₋₄₎-C(O)NR^{a}R^{b}, a C₁₋₄ alkyl-C₅₋₁₀ aryl group, a C₁₋₄ alkyl-5 to 10 membered N-containing heteroaryl group, a C₁₋₄ alkyl-5 to 10 membered N-containing heterocyclic group, a sulfonyl-C₅₋₁₀ aryl group, a sulfonyl-C₁₋₄ alkyl group,
wherein the aryl, heteroaryl and heterocyclic groups are optionally substituted by one or more substituents selected from the group consisting of a C₁₋₄ alkyl group, a C₁₋₄ alkoxy group, a hydroxy group, a halogen atom, a trifluoromethyl group, a carboxy group, a C₁₋₄ alkoxycarbonyl group, a thio-C₁₋₄ alkyl group, a sulfonyl-C₁₋₄ alkyl group, a cyano group, a carbamoyl group, -NR^{a}R^{b} and-C(O)NR^{a}R^{b}; and
wherein R^{a} and R^{b} independently represent a hydrogen atom or a C₁₋₄ alkyl group, and
• R³ is selected from the group consisting of a C₅₋₁₀ aryl group and a 5 to 10 membered heteroaryl group containing at least one heteroatom selected from N, S and O, wherein the aryl and the heteroaryl group optionally are substituted by one or more substituents selected from halogen atoms, C₁₋₄ alkyl groups and C₁₋₄ alkoxy groups,
or a pharmaceutically acceptable salt or N-oxide thereof.

2. A compound according to claim 1, wherein R¹ represents hydroxy group.

3. A compound according to claim 1 or 2, wherein R² represents hydrogen atom, a C₁₋₄ alkyl-C₅₋₁₀ aryl group, a C₁₋₄ alkyl-5 to 10 membered N-containing heteroaryl group, a C₁₋₄ alkyl-5 to 10 membered N-containing heterocyclic group, wherein the aryl, heteroaryl and heterocyclic groups are optionally substituted by one or two substituents selected from the group consisting of C₁₋₄ alkoxy group, carboxy group, C₁₋₄ alkoxycarbonyl group, cyano group and carbamoyl group.

4. A compound according to claim 3, wherein R² represents hydrogen atom, a C₁₋₄ alkyl-phenyl group, wherein the phenyl group is monosubstituted by a substituent selected from the group consisting of carboxy group, C₁₋₄ alkoxycarbonyl group, cyano group and carbamoyl group.

5. A compound according to claim 4, wherein R² represents a C₁₋₄ alkyl-phenyl group,
wherein the phenyl group is monosubstituted by a substituent selected from the group consisting of carboxy group, methoxycarbonyl group, cyano group and carbamoyl group.

6. A compound according to claims 1 to 5, wherein R³ represents a 5 to 10 membered heteroaryl group optionally substituted by one or two substituents selected from fluorine atoms, chlorine atoms and methyl groups.

7. A compound according to claim 6, wherein R³ represents a pyridyl group substituted by two chlorine atoms.

8. A compound according to claims 1 to 7, wherein:
• R¹ represents a hydroxy group;
• R² represents a C₁₋₄ alkyl-phenyl group, wherein the phenyl group is monosubstituted by a substituent selected from the group consisting of carboxy group, methoxycarbonyl group, cyano group and carbamoyl group; and
• R³ represents a pyridyl group substituted by two chlorine atoms.

9. A compound according to claim 1, wherein
• R¹ represents a hydroxy group or a amino group;
• R² represents a hydrogen atom, a methyl group, a -CH₂CONH₂ group, a pyridine-3-yl-methyl group, a benzyl group optionally substituted by one substitutent selected from the group consisting of a methoxy group, a carboxy group, a methoxycarbonyl group, a carbamoyl group and a cyano group; a 3-phenylpropyl group optionally substituted by one substituent selected from the group consisting of a cyano group and carbamoyl group; a 3-morpholinopropyl group or a 3-(dimethylcarbamoyl)phenylsulfonyl group;
• R³ represents a 3,5-dichloropyridin-4-yl group.

10. A compound according to claim 1 which is one of:
5-(3,5-Dichloropyridin-4-ylamino)-8,8-dimethyl-6,7,8,9-tetrahydro-3H-pyrazolo[3,4-c]isoquinolin-1-ol;
N⁵-(3,5-Dichloropyridin-4-yl)-8,8-dimethyl-6,7,8,9-tetrahydro-3H-pyrazolo[3,4-c]isoquinoline-1,5-diamine;
3-(5-(3,5-Dichloropyridin-4-ylamino)-1-hydroxy-8,8-dimethyl-6,7,8,9-tetrahydro-3H-pyrazolo[3,4-c]isoquinolin-3-ylsulfonyl)-N,N-dimethylbenzamide;
5-(3,5-Dichloropyridin-4-ylamino)-3-(3-methoxybenzyl)-8,8-dimethyl-6,7,8,9-tetrahydro-3H-pyrazolo[3,4-c]isoquinolin-1-ol;
5-(3,5-Dichloropyridin-4-ylamino)-3,8,8-trimethyl-6,7,8,9-tetrahydro-3H-pyrazolo[3,4-c]isoquinolin-1-ol;
Methyl 3-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid;
5-(3,5-Dichloropyridin-4-ylamino)-8,8-dimethyl-3-(pyridin-3-ylmethyl)-6,7,8,9-tetrahydro-3H-pyrazolo[3,4-c]isoquinolin-1-ol;
2-(5-(3,5-Dichloropyridin-4-ylamino)-1-hydroxy-8,8-dimethyl-6,7,8,9-tetrahydro-3H-pyrazolo[3,4-c]isoquinolin-3-yl)acetamide;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide;
Methyl 4-((5-(3,5-dichloropyridin-4-ylamino)-1-hydroxy-8,8-dimethyl-6,7,8,9-tetrahydro-3H-pyrazolo[3,4-c]isoquinolin-3-yl)methyl)benzoate
4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzoic acid;
3-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzonitrile;
4-((5-(3,5-Dichloropyridin-4-ylamino)-1-hydroxy-8,8-dimethyl-6,7,8,9-tetrahydro-3H-pyrazolo[3,4-c]isoquinolin-3-yl)methyl)benzamide;
5-(3,5-Dichloropyridin-4-ylamino)-8,8-dimethyl-3-(3-morpholinopropyl)-6,7,8,9-tetrahydro-3H-pyrazolo[3,4-c]isoquinolin-1-ol;
3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzonitrile;
3-(3-(5-(3,5-Dichloropyridin-4-ylamino)-1-hydroxy-8,8-dimethyl-6,7,8,9-tetrahydro-3H-pyrazolo[3,4-c]isoquinolin-3-yl)propyl)benzamide;
or a pharmaceutically acceptable salt or N-oxide thereof.

11. A compound according to any one of claims 1 to 10 for use in the treatment of a pathological condition or disease susceptible to amelioration by inhibition of phosphodiesterase IV.

12. A compound according to claim 11, wherein the pathological condition or disease is selected from asthma, chronic obstructive pulmonary disease, allergic rhinitis, rheumatoid arthritis, multiple sclerosis, atopic dermatitis, psoriasis or inflammatory bowel disease.

13. A pharmaceutical composition comprising a compound as defined in any one of claims 1 to 10 in association with a pharmaceutically acceptable diluent or carrier.

14. Use of a compound as defined in any one of claims 1 to 10 for the manufacture of a medicament for the treatment of a pathological condition or disease as defined in claims 11 or 12.

15. A method for treating a subject afflicted with a pathological condition or disease as defined in claims 11 or 12, which comprises administering to said subject an effective amount of a compound as defined in any one of claims 1 to 10.

16. A combination product comprising (i) a compound according to anyone of claims 1 to 10, and (ii) one or more active ingredients selected from the group consisting of β2-adrenergic agonist, anti-cholinergic, an anti-allergic agent, an anti-inflammatory agent, an immunosuppressant, and an anti-infective agent, for simultaneous, separate or sequential use in the treatment of the human or animal body.
